# EUROPEAN PATENT SPECIFICATION

(11) **EP 0 371 789 B1**
(45) Date of publication and mention of the grant of the patent: **02.01.1997**
(21) Application number: 89312437.0
(22) Date of filing: 29.11.1989
(51) Int. Cl.: A61K 31/19, A61K 31/195, A61K 31/22, A61K 31/41, A61K 31/185, C12Q 1/02, G01N 33/72

(54) **Medicament for augmenting fetal hemoglobin**
Arzneimittel zur Erhöhung des Gehaltes an fötalem Hämoglobin
Médicament pour l'augmentation de l'hémoglobine foetale

(30) Priority: 29.11.1988 US 277151
(43) Date of publication of application: 06.06.1990
(73) Proprietor: CHILDREN'S HOSPITAL MEDICAL CENTER OF NORTHERN CALIFORNIA, Oakland, CA 94609 (US)
(72) Inventor: Perrine, Susan, Richmond, CA 94806 (US)
(74) Representative: Baldock, Sharon Claire

(56) References cited:
- US-A- 4 482 571
- US-A- 4 822 821
- BIOCHEMICAL & BIOPHYSICAL RESEARCH COMMUNICATIONS, vol. 148, no. 2, 29 October 1987, Academic Press Inc.; S.P. PERRINE et al., pp. 694-698
- PROCEEDINGS OF THE NATL. ACADEMY OF SCIENCES USA, vol. 85, November 1988, Washington, DC (US); S.P. PERINE et al., pp. 8540-8542
- BLOOD, vol. 72, no. 6, December 1988, Grune & Stratton Inc.; P. CONSTANTOULAKIS et al., pp. 1961-1967

## Description

The present invention is directed to a method for inhibiting or reversing the switching in vivo or in vitro from production of γ to β-globin, thus augmenting the production of fetal hemoglobin. In particular, the present invention is directed to a method for controlling the fetal hemoglobin switch by introducing into the bloodstream of the fetus or child or into erythroid cultures a compound of the formula I, described herein below.

### BACKGROUND OF THE INVENTION

Normal adult hemoglobin comprises four globin proteins, two of which are α proteins and two of which are β proteins. Diseases known as sickle cell syndromes are associated with disorders in the β chain of the hemoglobin. However, in mammals, and particularly in humans, during fetal development, the fetus produces a fetal hemoglobin which comprises, instead of β-globin proteins, two γ-globin proteins. At some point during fetal development or infancy, depending on the particular species and individual, there is a so-called globin switch wherein the erythrocytes in the fetus switch from making predominantly γ-globin to making predominantly β-globin. It has been observed, however, that increased levels of fetal hemoglobin (derived from γ-globin) ameliorate the severity of sickling disorders. It has also been observed that subjects heterozygous for hereditary persistence of fetal hemoglobin syndromes (HPFH) and sickling hemoglobin (Hbs) are clinically asymptomatic of sickle cell anemia. Also, infants with sickle cell anemia do not usually develop the symptoms of the disease until approximately four months of age when their fetal hemoglobin levels decrease. These observations suggest that a method for increasing the levels of fetal hemoglobin would be beneficial to patients with sickle cell syndromes.

It is thus an object of the present invention to enable inhibiting or reversing the γ to β-globin switch in a fetus or infant to maintain increased fetal hemoglobin levels in those individuals with sickle cell syndromes.

Since inhibition of the γ to β-globin gene switch has been observed in infants of diabetic mothers (IDM), it appears that disturbances in normal metabolites in the bloodstream might disrupt a normally timed and fixed gene-switch. Two metabolites, insulin and butyric acid, have been observed as being elevated in infants of diabetic mothers and have been tested for their effect on γ to β-globin switching in vitro as described in Biochemical and Biophysical Research Comm., 148 (2), pp. 694-98 (1987). This work however has not been conclusive as to which metabolite is responsible for inhibition of the switching. Some investigators have shown that factors in serum can affect the γ to β-globin switching, particularly around the time the switch occurs. In Proc. Natl. Acad. Sci.USA, 85, pp. 8540-42 (1988), the effect of butyrate on the switching from γ to β-globin in fetal lambs was investigated and significant inhibition of the switch was demonstrated provided the butyrate was administered at an early enough stage in fetal development. United States patent 4,822,821 refers to the use of sodium butyrate, α-amino-n-butyric acid and butyric acid in methods for inhibiting the γ to β-globin switch in mammals. Other than the '821 patent, there has been, however, heretofore no development of a non-toxic therapeutic method or natural agent to increase fetal hemoglobin in subjects with sickle cell disease.

It is thus another object of the present invention to provide an agent for maintaining a high level of γ chain synthesis (thereby maintaining high fetal hemoglobin levels), without toxicity and long term side effects, by using a physiologic factor rather than a chemotherapeutic agent.

The invention provides for use of a compound of the formula I: 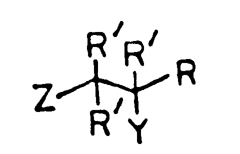 wherein R is -CO₂R₁, -SO₂R₁, -SO₃R₁, or 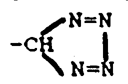 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or mixtures of these in the manufacture of a medicament for ameliorating β-globin disorders in a mammal;
provided that when R is -CO₂R₁ and Y is H, Z is -X or -CX₃.

The medicament is used in a method for ameliorating β-globin disorders in mammals comprising the step of periodically introducing into the bloodstream of said mammal during its gestation period and/or early infancy and thereafter, an amount of the compound described above sufficient to inhibit or reverse fetal γ to β-globin switching. The medicament according to the present invention is particularly useful for ameliorating in humans the clinical effects of sickle cell anemia.

In another aspect of the invention there is provided a method of determining the potential efficacy of further treatment for a β-globin disorder which method comprises the steps of:-
(a) culturing in vitro a cell sample taken from a mammal suffering from a β-globin disorder which has received treatment for said disorder,
(b) adding to said cultured sample a compound or mixture of compounds as defined above and
(c) measuring the level of β-globin and/or γ-globin produced by said culture to establish whether further treatment needs to be administered to the mammal.

The present invention permits a method for ameliorating the clinical effects of β-globin disorders, particularly the disorder of sickle cell anemia and β-thalassemias. The present invention is advantageous in that the compounds utilized are physiologic factors, i.e., natural metabolites found in the bloodstream of mammals and are thus, when introduced into the bloodstream, not likely to have toxic or undesirable long term side effects.

In accordance with the present invention, a compound of the formula I or a mixture of two or more thereof, are introduced into the bloodstream of the subject shown to or suspected of having a β-globin disorder, such as sickle cell anemia or β-thalassemias. The foregoing may be administered as their non-toxic salts, such as their sodium salts, ammonium salts, potassium salts, and the like. Preferred compounds are β-chloro-D-alanine, 3-chloropropionic acid, 5-(2-chloroethyl)tetrazole, heptafluorobutyric acid, α-amino propane sulfonic acid and sodium propanesulfinate.

Methods used to introduce the compound will be any convenient method normally used to introduce pharmaceuticals into the bloodstream, such as by injection or infusion, catheter, syringe, trans-cutaneous patch, in-dwelling depot delivery systems, and the like. Oral or parenteral administration may also be utilized.

The exact size of an effective dose of a compound according to the method of the present invention will depend on a number of factors including the particular recipient and the severity of condition, thus the route of administration will be ultimately at the discretion of the attendant physician.

While it is possible to utilize the compounds in vivo as a raw chemical, it is preferable to present them as a pharmaceutical formulation. The formulation of the present invention comprises a compound as previously described together with one or more acceptable carriers therefor and, optionally other therapeutic ingredients. The carriers must be acceptable in the sense of being compatible with other ingredients of the formulation and not dilatory as to the recipient.

For oral administration, the formulations may be presented as discrete units such as capsules, or tablets each containing a predetermined amount of the active ingredient, as a powder or granules, as a solution or suspension in an aqueous or nonaqueous liquid, as an oil in water liquid emulsion or as a water in oil liquid emulsion.

Formulations for parenteral administration include aqueous or nonaqueous sterile injection solutions which may contain antioxidants, buffers, bacteriostats and solutes which render the formulation isotonic with the blood of the intended recipient. The formulation may be presented in dose unit or multi-dose containers such as ampules or vials and may be stored in a freeze-dried condition requiring only the addition of a sterile liquid carrier, for example water, for injections.

Preferred unit dosage formulations are those containing a daily dose or a unit daily subdose, or an appropriate fraction thereof.

As further application of the compounds according to the present method, they may be utilized in vitro in cell cultures taken from patients to determine the potential efficacy of further treatment for the β-globin disorders, such as sickle cell anemia. The compounds may be thus used in vitro in cell cultures from patients to determine whether further addition of one of the compounds would result in continued inhibition or reversal of the globin switch.

The frequency and dosages of administration of the above compounds will depend upon when the compound is introduced, whether the subject is a fetus or an infant, the size and weight of the subject, the condition of the pregnant mother, and the like. Generally, injections beginning at a dosage as low as about 12 mg/kg body weight per day during gestation, particularly prior to the thirty-second week of gestation in humans, will delay the γ to β switching. Typically, larger dosages in the range of 500-1000 mg/kg of estimated fetal weight will be useful. Higher dosages may be utilized at the discretion of the physician. Since apparently the switching process is not complete in humans until approximately four months after birth, treatment may be preferentially initiated after birth up until about the fourth month of infancy and continued as long as necessary to maintain enhanced fetal hemoglobin levels in the patient. However, initiation of treatment even subsequent to the fourth month may also be effective.

All treatment should preferably occur prior to the fourth month of infancy since the γ to β switching process is difficult to reverse. Although treatment with one of the above compounds prior to the fourth month of infancy will inhibit the γ to β-switching process, treatment subsequent to that period may also achieve the desired clinical results, i.e., the amelioration of the effects of the β-globin disorder. Therefore, if the switching process is inhibited even to the extent of 10 to 20% (that is, the subject makes 10 to 20% more γ-globin than would be expected if the switch were allowed to occur), this may be sufficient to ameliorate the symptoms of the disease.

The method according to the present invention may be utilized in vivo, or in vitro as a diagnostic test. For the in vitro test, erythroid cultures, such as those obtained from cord blood mononuclear cells in Iscove's Modified Dulbecco's Medium with 0.9% methylcellulose, may be used as described by Stamatoyannopoulos et al., Blood, 54, 440-450 (1979) and Friedman et al., J. Clin. Invest., 75, 1359-1368 (1985). The cultures may be formed with and without the addition of various metabolites whose concentrations are observed to increase in infants of diabetic mothers (IDM). To show the effect of such factors, insulin (0.1-100 ng/ml), Insulin Growth Factor I (4-10 ng/ml), Isoleucine (4-10 mcg/ml), and butyric acid (0.01-10 mM) were added to the cultures. Some cultures were performed in 95% nitrogen to simulate a hypoxic intrauterine environment, and IDM plasma was used instead of fetal calf serum in some cultures. Colonies were harvested and incubated with ³H-leucine under the same culture conditions for the last 24 hours. Globin production was analyzed by electrophoresis on Triton-urea gels, by autoradiography, and by densitometry. In addition, radioligand immunoassays were performed as described in Stamatoyannopoulos et al., and Friedman et al. to determine the total and fetal hemoglobin per Bfu (Burst forming unit-erythroid) derived cell. In 10 Cfu-e (colony-forming unit-erythroid) cultures from five normal infants and five term IDM, mean β-globin synthesis (expressed as percent of total non-α synthesis) was 42.3% β-globin and 17.3% respectively. This is equivalent to the β-globin levels produced in infants' reticulocytes. This difference in percent β-globin synthesis between the IDM and normal infant Cfu-e was statistically significant (p<.001, Student's t-test) and indicates that these relatively late erythroid progenitors are committed to a pattern of globin production in the fetal environment. The percentage of β-globin synthesized by Bfu-e (burst-forming unit-erythroid) from normal term infants did not differ statistically from the percentage produced by their Cfu-e. However, Bfu-e from IDM produced a significantly higher percentage of the β-globin than did the infants'Cfu-e and reticulocytes (p<.05). This difference indicates that switching to increased β-globin production occurred once the more primitive progenitors were removed from the abnormal intrauterine environment or that the process began in vivo. Thus, the apparent in vitro switching by Bfu-e cultures from premature infants of less than 33 weeks gestation and from term IDM (pre-switch infants) is an assay system for testing the effect on globin switching of physiological parameters peculiar to the diabetic intrauterine environment, in which the globin switch is delayed. The addition of insulin, IGF-I, isoleucine, or low oxygen conditions causes the Bfu-e to synthesize the equivalent or more β-globin than control cultures.

Sodium butyrate (at 0.1 mM concentrations), caused Bfu-e from pre-switch infants to synthesize significantly less β-globin than did Bfu-e in control cultures. This effect of sodium butyrate was not found consistently in infants in whom the switch had occurred before birth. However, increased γ-globin synthesis was found consistently with α-amino-n-butyric acid. A significant increase in accumulation of hemoglobin F in the cord blood erythroid colonies also occurred in nine out of eleven cultures from seven infants, grown in the presence of sodium-butyrate compared to control cultures (p<.05). This effect on increasing hemoglobin F in culture was not due to toxicity since the pH of the culture media was not affected, and similar numbers of Bfu-e and Cfu-e were found in cultures grown in the presence or absence of the butyrates. Radioimmunoassay of Bfu-e hemoglobin indicates complete hemoglobinization (>15 pg/cell) in the presence of butyrate, demonstrating that the observed increase in hemoglobin F synthesis and accumulation were not an artifact of retardation of globin production.

The following examples are provided by way of illustration, however, the invention is not intended to be limited in any manner thereby.

### EXAMPLE 1

Cultures of erythroid progenitors from blood obtained during the first three years of life of Hb SS (sickle cell syndrome) infants are tested. It is found that β-chloro-D-alanine, 3-chloropropionic acid, 5-(2-chloroethyl) tetrazole, heptafluorobutyric acid, α-aminopropane sulfonic acid, or sodium propane sulfinate, increase γ-globin synthesis in two-thirds of infant cord blood erythroid colonies, SS samples from infants, even when the infants' reticulocytes and control colonies (Bfu-e) synthesize nearly all β-globin.

### EXAMPLE 2

Three fetal sheep are treated with continuous infusions of heptafluorobutyric acid in utero and the fetal to adult (β)hemoglobin switching process is delayed. In all three sheep, decrease in β-globin is observed within three days, thus evidencing a reversal of the switch. This effect persists for 5 days off treatment.

## Claims (Claims for the following Contracting State(s): GB, IT)

1. The use of a compound of the formula I: 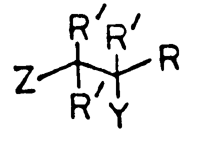 wherein R is -CO₂R₁, -SO₂R₁, -SO₃R₁, or 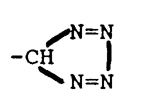 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, in the manufacture of a medicament for ameliorating β-globin disorders in a mammal;
provided that when R is -CO₂R₁ and Y is H, Z is -X or -CX₃.

2. The use according to claim 1, wherein said mammal is human and said disorder is sickle cell anemia or β-thalassemia.

3. The use according to claim 1 or 2, wherein said compound is selected from β-chloro-D-alanine, 3-chloropropionic acid, 5-(2-chloroethyl)tetrazole, heptafluorobutyric acid, α-aminopropanesulfonic acid, sodium propanesulfinate and mixtures thereof.

4. The use according to any one of claims 1 to 3, wherein said medicament is introduced into the bloodstream to the extent that γ-globin synthesis in said mammal remains at 10 to 20% above normal levels after birth.

5. A compound of the formula I: 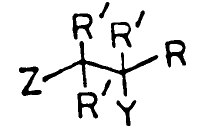 wherein R is -CO₂R₁, -SO₂R₁, -SO₃R₁, or 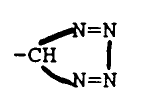 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, for use in ameliorating β-globin disorders in a mammal;
provided that when R is -CO₂R₁ and Y is H, Z is -X or -CX₃.

6. A pharmaceutical formulation for use in ameliorating β-globin disorders in a mammal which comprises a compound of the formula I: 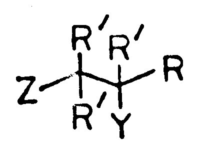 wherein R is -CO₂R₁, -SO₂R₁, -SO₃R₁, or 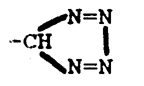 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, provided that when R is -CO₂R₁ and Y is H, Z is -X or -CX₃ and
a pharmaceutically acceptable carrier therefor.

7. A method of determining the potential efficacy of further treatment for a β-globin disorder, comprising the steps of:
(a) culturing in vitro a cell sample taken from a mammal suffering from a β-globin disorder which has received treatment for said disorder,
(b) adding to said cultured sample a compound or mixture of compounds as defined in claim 1, and
(c) measuring the level of β-globin, γ-globin or both, produced by said culture to establish whether further treatment should be administered to the mammal.

8. A process for making a pharmaceutical formulation for the amelioration of β-globin disorders in a mammal which comprises combining a compound of the formula I: 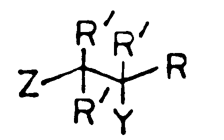 wherein R is -CO₂R₁, -SO₂R₁, -SO₃R₁, or 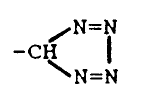 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, provided that when R is -CO₂R₁ and Y is H, Z is -X or -CX₃, with a pharmaceutically acceptable carrier therefor.

## Claims (Claims for the following Contracting State(s): GR)

1. The use of a compound of the formula I: 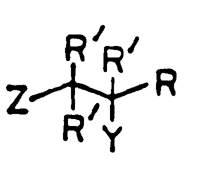 wherein R is -CO₂R₁, -SO₂R₁, -SO₃R₁, or 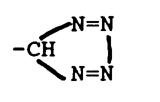 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, in the manufacture of a medicament for ameliorating β-globin disorders in a mammal;
provided that when R is -CO₂R₁ and Y is H, Z is -X or -CX₃.

2. The use according to claim 1, wherein said mammal is human and said disorder is sickle cell anemia or β-thalassemia.

3. The use according to claim 1 or 2, wherein said compound is selected from β-chloro-D-alanine, 3-chloropropionic acid, 5-(2-chloroethyl)tetrazole, heptafluorobutyric acid, α-aminopropanesulfonic acid, sodium propanesulfinate and mixtures thereof.

4. The use according to any one of claims 1 to 3, wherein said medicament is introduced into the bloodstream to the extent that γ-globin synthesis in said mammal remains at 10 to 20% above normal levels after birth.

5. A compound of the formula I: 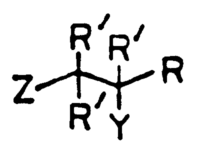 wherein R is -CO₂R₁, -SO₂R₁, -SO₃R₁, or 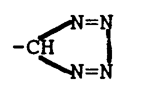 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, for use in ameliorating β-globin disorders in a mammal;
provided that when R is -CO₂R₁ and Y is H, Z is -X or -CX₃.

6. A method of determining the potential efficacy of further treatment for a β-globin disorder, comprising the steps of:
(a) culturing in vitro a cell sample taken from a mammal suffering from a β-globin disorder which has received treatment for said disorder,
(b) adding to said cultured sample a compound or mixture of compounds as defined in claim 1, and
(c) measuring the level of β-globin, γ-globin or both, produced by said culture to establish whether further treatment should be administered to the mammal.

7. A process for making a pharmaceutical formulation for the amelioration of β-globin disorders in a mammal which comprises combining a compound of the formula I: 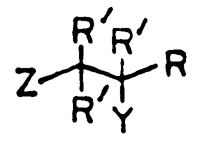 wherein R is -CO₂R₁, -SO₂R₁, -SO₃R₁, or 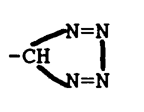 and R₁ is H, M, or alkyl 1-4 carbons and M is a cation; Z is -CH₃, -X, or -CX₃; and X is Cl, F, Br, or I; Y is H, -NH₂, -NH₃, or F; and R' is H or F; or a mixture thereof, provided that when R is -CO₂R₁ and Y is H, Z is -X or -CX₃, with a pharmaceutically acceptable carrier therefor.

## Patentansprüche (Patentansprüche für folgende(n) Vertragsstaat(en): GB, IT)

1. Verwendung einer Verbindung nach Formel I: 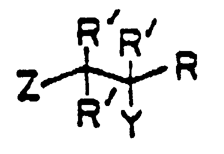 worin R -CO₂R₁, -SO₂R₁, -SO₃R₁ oder 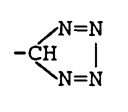 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder eines Gemisches davon bei der Herstellung eines Medikaments zur Besserung von β-Globin Krankheiten in einem Säuger, unter der Maßgabe, daß, wenn R -CO₂R₁ ist und Y H ist, Z -X oder -CX₃ ist.

2. Verwendung nach Anspruch 1, wobei der Säuger ein Mensch ist und die Krankheit Sichelzellanämie oder β-Thalassämie ist.

3. Verwendung nach Anspruch 1 oder 2, wobei die Verbindung ausgewählt wird aus β-Chlor-D-alanin, 3-Chlorpropionsäure, 5-(2-Chlorethyl)tetrazol, Heptafluorbuttersäure, α-Aminopropansulfonsäure, Natriumpropansulfinat und Gemischen davon.

4. Verwendung nach einem der Ansprüche 1 bis 3, wobei das Medikament in dem Umfang in den Blutstrom eingebracht wird, daß die γ-Globin Synthese in dem Säuger bei 10 bis 20% oberhalb normaler Werte nach der Geburt bleibt.

5. Verbindung nach Formel I: 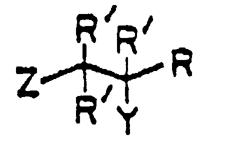 worin R -CO₂R₁, -SO₂R₁, -SO₃R₁ oder 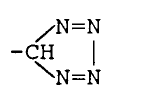 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder ein Gemisch davon zur Verwendung für die Besserung von β-Globin Krankheiten in einem Säuger, unter der Maßgabe, daß, wenn R -CO₂R₁ ist und Y H ist, Z -X oder -CX₃ ist.

6. Pharmazeutische Formulierung zur Verwendung für die Besserung von β-Globin Krankheiten in einem Säuger, umfassend eine Verbindung nach Formel I: 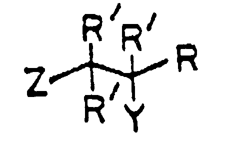 worin R -CO₂R₁, -SO₂R₁, -SO₃R₁ oder 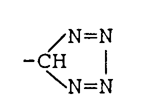 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder ein Gemisch davon, unter der Maßgabe, daß, wenn R -CO₂R₁ ist und Y H ist, Z -X oder -CX₃ ist und einen pharmazeutisch verträglichen Träger dafür.

7. Verfahren zum Bestimmen der potentiellen Wirksamkeit weiterer Behandlung für eine β-Globin Krankheit, umfassend die Schritte:
(a) in vitro Kultivieren einer Zellprobe, die einem an einer β-Globin Krankheit leidenden Säuger, welcher eine Behandlung für die Krankheit erhalten hat, entnommen wurde,
(b) Zugeben einer Verbindung oder eines Gemisches von Verbindungen nach Anspruch 1 zu der kultivierten Probe, und
(c) Bestimmen des von der Kultur erzeugten Gehalts von β-Globin, γ-Globin oder von beiden um festzustellen, ob dem Säuger weitere Behandlung verabreicht werden sollte.

8. Verfahren zur Herstellung einer pharmazeutischen Formulierung zur Besserung von β-Globin Krankheiten in einem Säuger, umfassend das Vereinigen einer Verbindung nach Formel I: 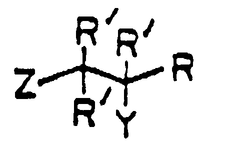 worin R -CO₂R₁, -SO₂R₁, -SO₃R₁ oder 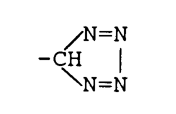 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder eines Gemisches davon, unter der Maßgabe, daß, wenn R -CO₂R₁ ist und Y H ist, Z -X oder -CX₃ ist, mit einem pharmazeutisch verträglichen Träger dafür.

## Patentansprüche (Patentansprüche für folgende(n) Vertragsstaat(en): GR)

1. Verwendung einer Verbindung nach Formel I: 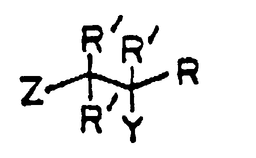 worin R -CO₂R₁, -SO₂R₁, -SO₃R₁ oder 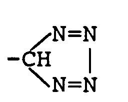 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder eines Gemisches davon bei der Herstellung eines Medikaments zur Besserung von β-Globin Krankheiten in einem Säuger, unter der Maßgabe, daß, wenn R -CO₂R₁ ist und Y H ist, Z -X oder -CX₃ ist.

2. Verwendung nach Anspruch 1, wobei der Säuger ein Mensch ist und die Krankheit Sichelzellanämie oder β-Thalassämie ist.

3. Verwendung nach Anspruch 1 oder 2, wobei die Verbindung ausgewählt wird aus β-Chlor-D-alanin, 3-Chlorpropionsäure, 5-(2-Chlorethyl)tetrazol, Heptafluorbuttersäure, α-Aminopropansulfonsäure, Natriumpropansulfinat und Gemischen davon.

4. Verwendung nach einem der Ansprüche 1 bis 3, wobei das Medikament in dem Umfang in den Blutstrom eingebracht wird, daß die γ-Globin Synthese in dem Säuger bei 10 bis 20% oberhalb normaler Werte nach der Geburt bleibt.

5. Verbindung nach Formel I: 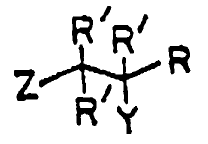 worin R -CO₂R₁, -SO₂R₁, -SO₃R₁ oder 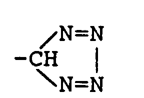 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder ein Gemisch davon zur Verwendung für die Besserung von β-Globin Krankheiten in einem Säuger, unter der Maßgabe, daß, wenn R -CO₂R₁ ist und Y H ist, Z -X oder -CX₃ ist.

6. Verfahren zum Bestimmen der potentiellen Wirksamkeit weiterer Behandlung für eine β-Globin Krankheit, umfassend die Schritte:
(a) in vitro Kultivieren einer Zellprobe, die einem an einer β-Globin Krankheit leidenden Säuger, welcher eine Behandlung für die Krankheit erhalten hat, entnommen wurde,
(b) Zugeben einer Verbindung oder eines Gemisches von Verbindungen nach Anspruch 1 zu der kultivierten Probe, und
(c) Bestimmen des von der Kultur erzeugten Gehalts von β-Globin, γ-Globin oder von beiden um festzustellen, ob dem Säuger weitere Behandlung verabreicht werden sollte.

7. Verfahren zur Herstellung einer pharmazeutischen Formulierung zur Besserung von β-Globin Krankheiten in einem Säuger, umfassend das Vereinigen einer Verbindung nach Formel I: 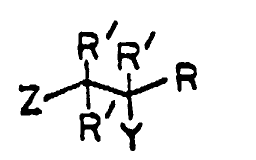 worin R -CO₂R₁, -SO₂R₁, -SO₃R₁ oder 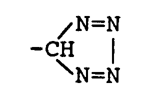 ist und R₁ H, M oder C₁-C₄ Alkyl ist und M ein Kation ist, Z -CH₃, -X oder -CX₃ ist und X Cl, F, Br oder I ist, Y H, -NH₂, -NH₃ oder F ist und R' H oder F ist, oder eines Gemisches davon, unter der Maßgabe, daß, wenn R -CO₂R₁ ist und Y H ist, Z -X oder -CX₃ ist, mit einem pharmazeutisch verträglichen Träger dafür.

## Revendications (Revendications pour l'(les) Etat(s) contractant(s) suivant(s): GB, IT)

1. Utilisation d'un composé de formule I : 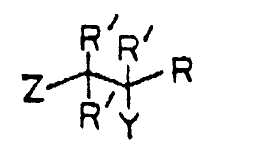 dans laquelle R représente -CO₃R₁, -SO₂R₁, -SO₃R₁ ou 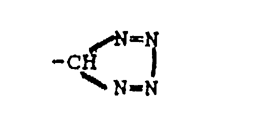 et R₁ représente H, M ou un groupe alkyle ayant 1 à 4 atomes de carbone, et M représente un cation ; Z représente -CH₃, -X ou -CX₃ ; et X représente Cl, F, Br ou I ; Y représente H, -NH₂, -NH₃ ou F ; et R' représente H ou F ; ou d'un mélange de ces composés, dans la fabrication d'un médicament pour améliorer l'état d'un mammifère souffrant de troubles de la β-globine,
à la condition que, lorsque R représente -CO₂R₁ et que Y représente H, Z représente -X ou -CX₃.

2. Utilisation selon la revendication 1, dans laquelle ledit mammifère est un être humain et ledit trouble est de l'anémie à hématies falciformes ou de la β-thalassémie.

3. Utilisation selon la revendication 1 ou 2, dans laquelle ledit composé est choisi parmi la β-chloro-D-alanine, l'acide 3-chloropropionique, le 5-(2-chloroéthyl)tétrazole, l'acide heptafluorobutyrique, l'acide α-aminopropanesulfonique, le propanesulfinate de sodium et leurs mélanges.

4. Utilisation selon l'une quelconque des revendications 1 à 3, dans laquelle ledit médicament est introduit dans le courant sanguin en une quantité telle que la synthèse de la γ-globine demeure dans ledit mammifère à des taux de 10 à 20 % au-dessus des taux normaux après la naissance.

5. Composé de formule I : 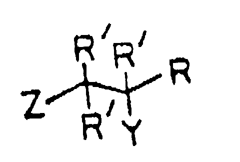 dans laquelle R représente -CO₂R₁, -SO₂R₁, -SO₃R₁ ou 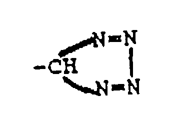 et R₁ représente H, M ou un groupe alkyle ayant 1 à 4 atomes de carbone et M représente un cation ; Z représente -CH₃, -X ou -CX₃ ; et X représente Cl, F, Br ou I ; Y représente H, -NH₂, -NH₃ ou F ; et R' représente H ou F ; ou d'un de leurs mélanges, à utiliser pour améliorer l'état d'un mammifère souffrant de troubles de la β-globine ;
à la condition que, lorsque R représente -CO₂R₁ et que Y représente H, Z représente -X ou -CX₃.

6. Formulation pharmaceutique à utiliser dans l'amélioration de l'état d'un mammifère souffrant de troubles de la β-globine, la formulation comprenant un composé de formule I : 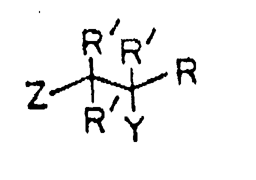 dans laquelle R représente -CO₂R₁, -SO₂R₁, -SO₃R₁ ou 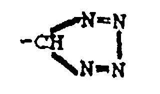 et R₁ représente H, M ou un groupe alkyle ayant 1 à 4 atomes de carbone, et M représente un cation ; Z repréprésente -CH₃, -X ou -CX₃ ; et X représente Cl, F, Br ou I ; Y représente H, -NH₂, -NH₃ ou F ; et R' représente H ou F ; ou un mélange de tels composés, à la condition que, lorsque R représente -CO₂R₁ et que Y représente H, Z représente -X ou -CX₃, et un véhicule ou excipient pharmaceutiquement acceptable pour ce ou ces composés.

7. Procédé pour déterminer l'efficacité potentielle de la poursuite du traitement d'un trouble de la β-globine, le procédé comprenant les étapes consistant à :
(a) cultiver in vitro un échantillon de cellules prélevé sur un mammifère souffrant d'un trouble de la β-globine, et qui a reçu un traitement pour ledit trouble ;
(b) ajouter audit échantillon cultivé un composé ou un mélange de composés selon la définition donnée à la revendication 1, et
(c) mesurer le taux de β-globine, de γ-globine ou des deux, produit par ladite culture pour établir s'il convient de continuer à administrer le traitement au mammifère.

8. Procédé pour produire une formulation pharmaceutique pour améliorer l'état d'un mammifère souffrant de troubles de la β-globine, ce procédé comprenant la combinaison d'un composé de formule I : 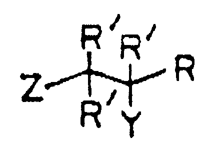 dans laquelle R représente -CO₂R₁, -SO₂R₁, -SO₃R₁ ou 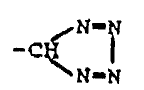 et R₁ représente H, M ou un groupe alkyle ayant 1 à 4 atomes de carbone et M représente un cation ; Z représente -CH₃, -X ou -CX₃ ; et X représente Cl, F, Br ou I ; Y représente H, -NH₂, -NH₃ ou F ; et R' représente H ou F ; ou d'un de leurs mélanges, à la condition que, lorsque R représente -CO₂R₁ et que Y représente H, Z représente -X - ou -CX₃, avec un véhicule pharmaceutiquement acceptable de ce ou de ces composés.

## Revendications (Revendications pour l'(les) Etat(s) contractant(s) suivant(s): GR)

1. Utilisation d'un composé de formule I : 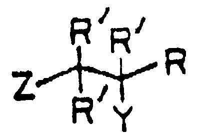 dans laquelle R représente -CO₂R₁, -SO₂R₁, -SO₃R₁, ou 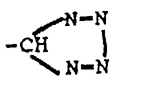 et R₁ représente H, M ou un groupe alkyle ayant 1 à 4 atomes de carbone et M représente un cation : Z représente -CH₃, -X ou -CX₃ ; et X représente Cl, F, Br ou I ; Y représente H, NH₂, -NH₃ ou F ; et R' représente H ou F ; ou d'un de leurs mélanges, dans la fabrication d'un médicament pour améliorer l'état de troubles de la β-globine chez un mammifère ;
à la condition que, lorsque R représente -CO₂R₁ et Y représente H, Z représente -X ou -CX₃.

2. Utilisation selon la revendication 1, dans laquelle ledit mammifère est un être humain et ledit trouble est de l'anémie à hématies falciformes ou de la β-thalassémie.

3. Utilisation selon la revendication 1 ou 2, dans laquelle le dit composé est choisi parmi la β-chloro-D-alanine, l'acide 3-chloropropionique, le 5-(2-chloroéthyl)tétrazole, l'acide heptafluorobutyrique, l'acide α-aminopropanesulfonique, le propanesulfinate de sodium et leurs mélanges.

4. Utilisation selon l'une des revendications 1 à 3, dans laquelle ledit médicament est introduit dans le courant sanguin dans une proportion telle que la synthèse de γ-globine dans ledit mammifère demeure à 10 à 20 % au-dessus des taux normaux après la naissance.

5. Composé de formule I : 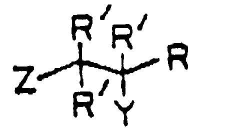 dans laquelle R représente -CO₂R₁, -SO₂R₁, -SO₃R₁ ou 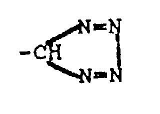 et R₁ représente H, M ou un groupe alkyle ayant 1 à 4 atomes de carbone et M représente un cation ; Z représente -CH₃, -X ou -CX₃ ; et X représente Cl, F, Br ou I ; Y représente H, -NH₂, -NH₃ ou F ; et R' représente H ou F ; ou un de leurs mélanges, à utiliser pour améliorer l'état d'un mammifère souffrant de troubles de β-globine,
à la condition que, lorsque R représente -CO₂R₁ et que Y représente H, Z représente -X ou -CX₃.

6. Procédé pour déterminer l'efficacité potentielle de la poursuite du traitement d'un trouble de β-globine, ce procédé comprenant les étapes consistant à :
(a) cultiver in vitro un échantillon de cellules prélevé sur un mammifère souffrant d'un trouble de β-globine, et qui a reçu un traitement pour ledit trouble,
(b) ajouter audit échantillon cultivé un composé, ou un mélange de composés, selon la définition de la revendication 1, et
(c) mesurer le taux de β-globine, de γ-globine ou des deux, produit par ladite culture pour établir s'il convient de poursuivre l'administration du traitement au mammifère.

7. Procédé pour produire une formulation pharmaceutique pour améliorer l'état d'un mammifère souffrant de troubles de la β-globine, procédé comprenant la combinaison d'un composé de formule I : 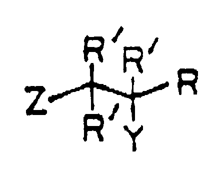 dans laquelle R représente -CO₂R₁, -SO₂R₁, -SO₃R₁ ou 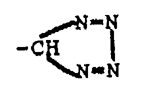 et R₁ représente H, M ou un groupe alkyle ayant 1 à 4 atomes de carbone et M représente un cation ; Z représente -CH₃, -X ou -CX₃ ; et X représente Cl, F, Br ou I ; Y représente H, -NH₂, -NH₃ ou F ; et R' représente H ou F ; ou un mélange de tels composés, à la conditions que, lorsque R représente -CO₂R₁ et que Y représente H, Z représente -X ou -CWX₃, avec un excipient pharmaceutiquement acceptable du ou des composés.
